(19) Europäisches Patentamt
European Patent Office
Office européen des brevets

(11) Veröffentlichungsnummer: **0 295 209**
**A2**

(12) # EUROPÄISCHE PATENTANMELDUNG

(21) Anmeldenummer: **88810360.3**

(22) Anmeldetag: **02.06.88**

(51) Int. Cl.⁴: **C 07 D 519/00**
C 08 K 5/15
//(C07D519/00,493:00,493:00)

(30) Priorität: **10.06.87 CH 2173/87**

(43) Veröffentlichungstag der Anmeldung:
**14.12.88 Patentblatt 88/50**

(84) Benannte Vertragsstaaten:
**CH DE ES FR GB IT LI NL**

(71) Anmelder: **CIBA-GEIGY AG**
**Klybeckstrasse 141**
**CH-4002 Basel (CH)**

(72) Erfinder: **Flury, Peter, Dr.**
**Muldenweg**
**CH-4249 Himmelried (CH)**

**Eldin, Sameer H., Dr.**
**Route de Schiffenen 10**
**CH-1700 Fribourg (CH)**

**Roth, Martin, Dr.**
**Oberdorf**
**CH-1711 Giffers (CH)**

**Rabener, Claus Willi, Dr.**
**Schmiedackerstrasse 21**
**D-7858 Oetlingen (DE)**

(54) **Orthokohlensäureester.**

(57) Orthokohlensäureester der Formel I

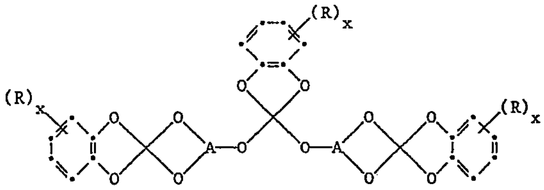

(I),

worin R $C_1$-$C_4$-Alkyl und x Null, 1 oder 2 bedeuten und A ein von einem aliphatischen Triol durch Weglassen der Hydroxylgruppen abgeleiteter dreiwertiger Rest ist, wobei die Hydroxylgruppen des Triols an verschiedene Kohlenstoffatome gebunden sind, eignen sich als Cokomponenten bei der Härtung von Epoxidharzen, indem sie eine Reduktion der während der Härtung von Epoxidharzen auftretenden Volumenverminderung verursachen. Die ausgehärteten Harze zeichnen sich zudem durch eine erhöhte Biegefestigkeit und Schlagzähigkeit aus.

EP 0 295 209 A2

**Beschreibung**

**Orthokohlensäureester**

Die Erfindung betrifft neue polycyclische Orthokohlensäureester, ein Verfahren zu deren Herstellung sowie deren Verwendung als Cokomponenten in härtbaren Epoxidharzstoffgemischen.

Bei der Härtung von Epoxidharzen tritt im allgemeinen eine Volumenverminderung auf, welche zu inneren Spannungen und Schwierigkeiten in der Formgebung führt. In der Praxis wird oft versucht, durch Zugabe von verschiedenen Zusätzen, unter anderem auch von bicyclischen Orthokohlensäureestern, diese Volumenabnahme möglichst zu reduzieren.

So beschreibt die US 4,387,215 ein Verfahren zur Herstellung von Polymeren ohne Volumenabnahme durch Verwendung von Spiroorthocarbonaten, Spiroorthoestern oder polycyclischen Ketal-Lactonen. Es wird zwar erwähnt, dass die verwendeten Spiro-bzw. polycyclischen Verbindungen auch zusammen mit anderen Monomeren zur Herstellung von Copolymeren mit reduzierter Volumenabnahme eingesetzt werden können, aber keine solchen Copolymere sind spezifisch offenbart.

Im ACS Polymeric Materials Science and Engineering, Band 54, Seite 23 beschreiben W.J. Bailey and Mitarbeiter die Härtung eines Bisphenol-A Diglycidylethers in Anwesenheit eines Bisnorbornenylspiroorthocarbonats sowie die Herstellung anderer bisaliphatischer oder bisaromatischer Spiroorthokohlensäureester.

Die bekannten Orthokohlensäureester vermögen aber bei ihrer Anwendung als Cokomponenten bei der Härtung von Epoxidharzen nicht in jeder Beziehung zufriedenzustellen. Viele dieser Verbindungen sind entweder mit den üblichen Epoxidharzen nicht in genügendem Ausmass mischbar oder sie beeinträchtigen die übrigen Eigenschaften der gehärteten Produkte, wie z.B. die Wärmeformbeständigkeit oder die Biegefestigkeit. Die erfindungsgemässen Verbindungen weisen die erwähnten Nachteile nicht auf.

Gegenstand der vorliegenden Erfindung sind Orthokohlensäureester der Formel I

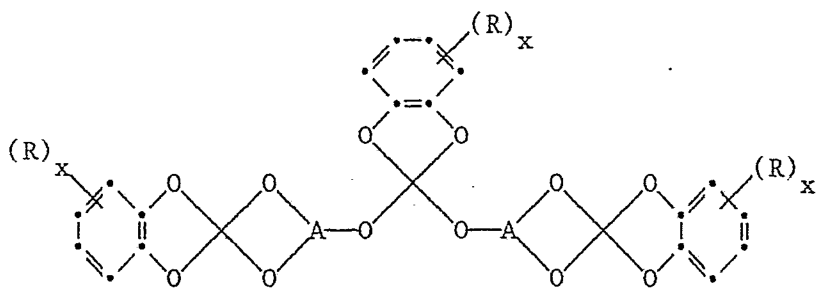

(I),

worin R $C_1$-$C_4$-Alkyl und x Null, 1 oder 2 bedeuten und A ein von einem aliphatischen Triol durch Weglassen der Hydroxylgruppen abgeleiteter dreiwertiger Rest ist, wobei die Hydroxylgruppen des Triols an verschiedene Kohlenstoffatome gebunden sind.

Die erfindungsgemässen Orthokohlensäureester können durch Umsetzung von drei Mol eines 2,2-Dichlor-1,3-benzodioxols der Formel

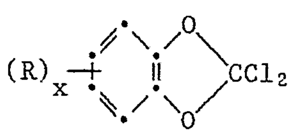

mit zwei Mol eines Triols der Formel $A(OH)_3$ in Gegenwart einer Base hergestellt werden, wobei die Symbole R, x und A die oben angegebene Bedeutung haben.

Bevorzugt werden Orthokohlensäureester der Formel I, worin x 1 und insbesondere Null bedeutet.

Bevorzugt sind auch Verbindungen der Formel I, worin A von einem 1,2,n-Triol, mit n gleich eine ganze Zahl von 3 bis 10, oder von einem 1,3,5-Triol abgeleitet ist. Als 1,2,n-Triole, von denen der Rest A abgeleitet sein kann, kommen insbesondere 1,2,3-, 1,2,4- und 1,2,5-Triole in Frage.

Besonders bevorzugt sind Verbindungen der Formel I, worin A von einem 1,2,3-, 1,2,4- oder von einem 1,3,5-Triol abgeleitet ist, sowie solche, worin A ein 3 bis 24, insbesondere 3 bis 12, Kohlenstoffatome enthaltender Rest ist.

Wenn A einen dreiwertigen, von einem 1,2,3-Triol abgeleiteten Rest bedeutet, ist es vorzugsweise ein Rest der Formel II

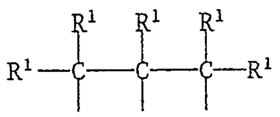 (II),

wobei die Substituenten $R^1$ unabhängig voneinander eine $C_1$-$C_4$-Alkylgruppe oder vorzugsweise Wasserstoff bedeuten.

Wenn die Gruppen R oder $R^1$ $C_1$-$C_4$-Alkylgruppen bedeuten, so können diese geradkettig oder verzweigt sein, wie z.B. Methyl, Ethyl, n-und i-Propyl sowie n-, i-, sec- und tert-Butyl.

Als 1,2,3-Triole von denen der Rest A abgeleitet sein kann, kommen z.B. die folgenden in Frage: Glycerin, 1,2,3-Butantriol, 1,2,3-Pentantriol, 2,3,4-Pentantriol, 3-Methyl-1,2,3-Butantriol, 1,2,3-Hexantriol, 2,3,4-Hexantriol und 2,4-Dimethyl-2,3,4-pentantriol.

Das am meisten bevorzugte 1,2,3-Triol ist Glycerin.

Als 1,2,4- oder 1,2,5-Triole, von denen der Rest A abgeleitet sein kann, kommen z.B. die folgenden in Frage: 1,2,4-Butantriol, 1,2,4-Pentantriol, 1,2,4-Hexantriol und 1,2,5-Hexantriol. 1,2,4-Butantriol ist dabei besonders bevorzugt.

Als 1,3,5-Triole, von denen der Rest A abgeleitet sein kann, kommen z.B. die folgenden Verbindungen in Frage: 1,1,1-Trimethylolpropan [2,2-Bis(hydroxymethyl)butanol], Trimethylolmethan und 1,1,1-Trimethylolethan.

Bevorzugt sind 1,1,1-Trimethylolpropan und 1,1,1-Trimethylolethan.

Bevorzugt sind somit auch erfindungsgemässe Orthokohlensäureester der Formel I, worin A ein Rest der Formel III, IIIa oder IIIb

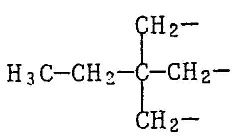

(III)

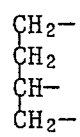

(IIIa)

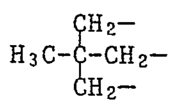 ist.

(IIIb)

Am meisten bevorzugt werden erfindungsgemässe Orthokohlensäureester der Formeln IV bis XI

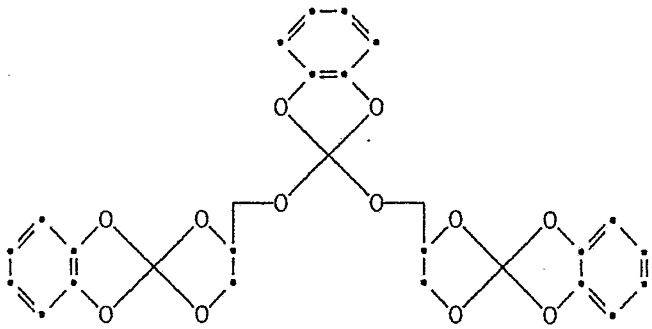

IV

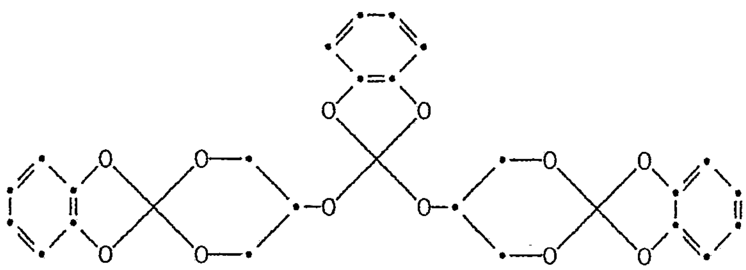

V

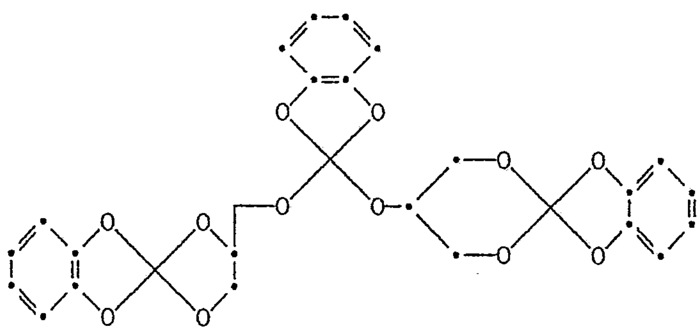

VI

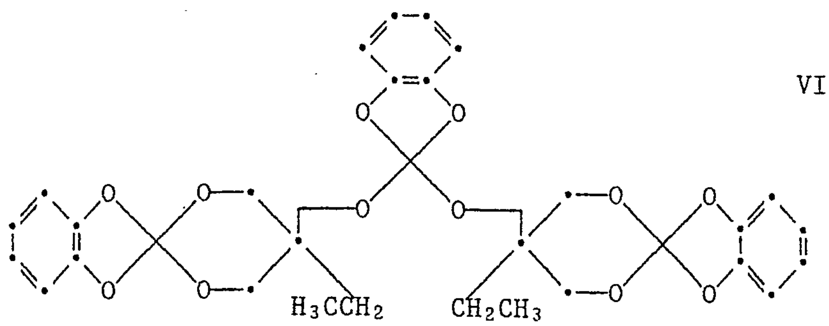

VII

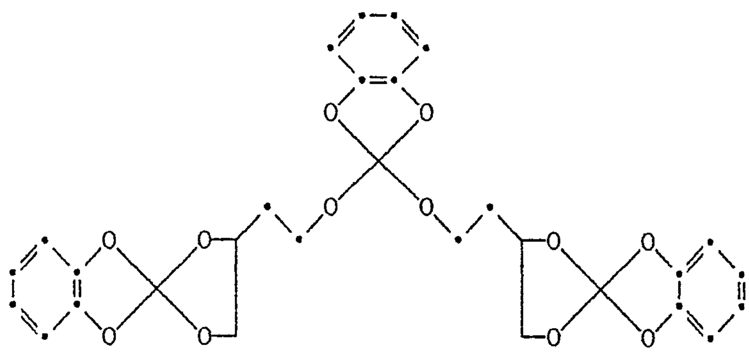

VIII

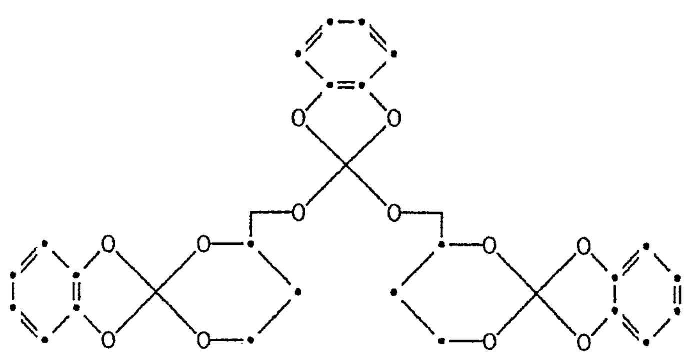

IX

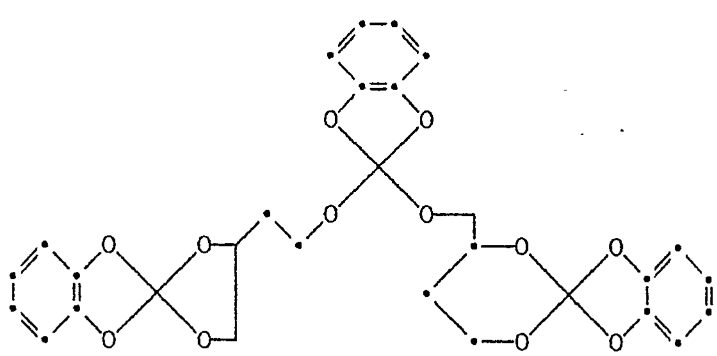

X

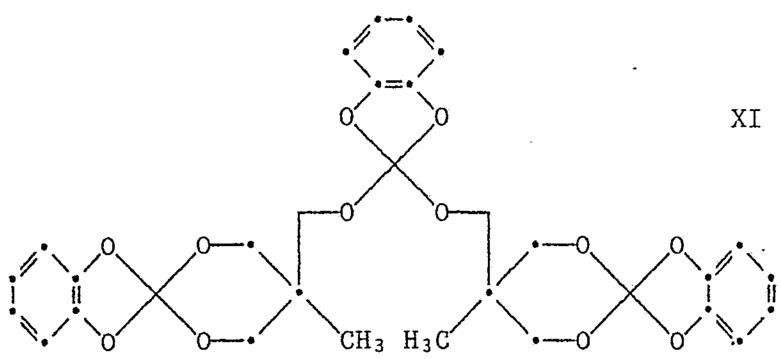

XI

Diese Verbindungen sind Umsetzungsprodukte von 2,2-Dichlor-1,3-benzodioxol mit Glycerin, mit Trimethylolpropan, mit 1,2,4-Butantriol bzw. mit Trimethylolethan. Bei der Umsetzung mit Glycerin entsteht vorwiegend die Verbindung der Formel IV, es können aber auch kleinere Mengen der isomeren Verbindungen der Formeln V und VI gebildet werden.

Wie gesagt, können die erfindungsgemässen Orthokohlensäureester durch Umsetzung von 3 Mol eines gegebenenfalls substituierten 2,2-Dichlor-1,3-benzodioxols mit 2 Mol eines Triols in Gegenwart einer Base hergestellt werden. Geeignete Basen sind insbesondere tertiäre Amine, wie z.B. Triethylamin oder Pyridin. Die Menge der Base sollte mindestens der Menge der durch die Umsetzung freigesetzten Säure entsprechen. Es kann aber auch ein grösserer Ueberschuss der Base verwendet werden, so dass diese auch zum Teil als Lösungsmittel dient. Die Umsetzung erfolgt vorzugsweise in einem inerten Lösungsmittel, wie z.B. Toluol, Methylenchlorid, Chloroform oder Essigester, falls zweckmässig, unter gleichzeitiger Kühlung des Reaktionsgemisches. Das bei der Umsetzung erhaltene Orthokohlensäureester-Produkt wird aus dem Reaktionsgemisch isoliert und kann direkt weiterverwendet werden, unabhängig davon, ob neben dem Hauptprodukt (z.B. der Verbindung der Formel IV) auch noch kleinere Mengen isomerer Nebenprodukte entstehen (z.B. Verbindungen der Formeln V und VI).

Die bei der Umsetzung verwendeten Ausgangsstoffe sind bekannt und können auf bekannte Weise hergestellt werden. Gegebenenfalls substituierte 2,2-Dichlor-1,3-benzodioxole können z.B., wie im Houben-Weyl, "Methoden der organischen Chemie", Bd. E4, 667-668, Thieme Verlag, Stuttgart 1983 beschrieben, durch Chlorierung von 1,3-Benzodioxolen oder von 2-Oxo-1,3- Benzodioxolen hergestellt werden. Die bei der Herstellung der erfindungsgemässen Verbindungen verwendeten Triole sind bekannt und sind im Handel erhältlich.

Die erfindungsgemässen Orthokohlensäureester sind mit Epoxidharzen in einem breiten Mengenverhältnis sehr gut mischbar und reagieren bei der Härtung mit dem Epoxidharz-Härter-System, um gehärtete Produkte mit vorzüglichen Eigenschaften zu ergeben.

Gegenstand der Erfindung sind auch härtbare Stoffgemische enthaltend

(a) einen erfindungsgemässen Orthokohlensäureester der Formel I,

(b) ein Epoxidharz und

(c) einen Härter und gegebenenfalls einen Härtungskatalysator für das Epoxidharz.

Die erfindungsgemässen härtbaren Stoffgemische enthalten vorzugsweise 5 bis 50, besonders bevorzugt 10 bis 40, insbesondere 15 bis 35 Gewichtsteile des Orthokohlensäureesters (a) pro 100 Gewichtsteile des Epoxidharzes (b).

Die erfindungsgemässen Orthokohlensäureester reduzieren nachhaltig die während der Härtung von Epoxidharzen üblicherweise auftretende Volumenverminderung, welche sonst die Eigenschaften der gehärteten Produkte beeinträchtigt. Gegenstand der Erfindung ist daher auch die Verwendung der Orthokohlensäureester der Formel I als Cokomponenten bei der Härtung von Epoxidharzen, zur Reduktion der während der Härtung von Epoxidharzen auftretenden Volumenverminderung.

Bei den Komponenten (a), (b) und (c) der erfindungsgemässen Stoffgemische kann es sich jeweils um reine Stoffe oder um Mischungen handeln.

In den erfindungsgemässen Stoffgemischen kommen als Komponente (b) alle üblichen Epoxidharze in Frage. Insbesondere seien genannt:

Alicyclische Polyepoxide, wie Epoxyethyl-3,4-epoxycyclohexan (Vinylcyclohexendiepoxid), Limonendiepoxid, Dicyclopentadiendiepoxid, Bis(3,4-epoxycyclohexylmethyl)adipat, 3',4'-Epoxycyclohexylmethyl- 3,4-epoxycyclohexancarboxylat, 3',4'-Epoxy-6'-methylcyclohexylmethyl-3,4-epoxy-6-methylcyclohexancarboxylat, 3-(3'-4'-Epoxycyclohexyl)-2,4-dioxaspiro[5,5]-8,9-epoxyundecan, 3-Glycidyloxyethoxyethyl-2,4-dioxaspiro[5,5]-8,9-epoxyundecan.

Di- oder Polyglycidylether von mehrwertigen Alkoholen, wie 1,4-Butandiol oder Polyalkylenglykolen, wie Polypropylenglykole, Di-oder Polyglycidylether von cycloaliphatischen Polyolen, wie 2,2-Bis(4-hydroxycyclohexyl)propan, Di- oder Polyglycidylether von mehrwertigen Phenolen, wie Resorcin, Bis(p-hydroxyphenyl)methan (Bisphenol F), 2,2-Bis(p-hydroxyphenyl)propan (Bisphenol A), 2,2-Bis(4'-hydroxy-3',5'-dibromphenyl)propan, 1,1,2,2-Tetrakis(p-hydroxyphenyl)ethan, oder unter saueren Bedingungen erhaltene Kondensationsprodukte von Phenolen mit Formaldehyd, wie Phenol-Novolake und Kresol-Novolake; ferner Di-oder Poly(β-methylglycidyl)ether der oben angeführten Polyalkohole und Polyphenole.

Polyglycidylester und Poly(β-methylglycidyl)ester von mehrwertigen Carbonsäuren, wie Phthalsäure, Terephthalsäure, Tetrahydrophthalsäure und Hexahydrophthalsäure.

N-Glycidylderivate von Aminen, Amiden und heterocyclischen Stickstoffbasen, wie N,N-Diglycidylanilin, N,N-Diglycidyltoluidin, N,N,N',N'-Tetraglycidyl-bis(p-aminophenyl)methan, Triglycidylisocyanurat, N,N'-Diglycidylethylenharnstoff, N,N'-Diglycidyl-5,5-dimethylhydantoin , N,N'-Diglycidyl-5-isopropyl-hydantoin, N,N'-Diglycidyl-5,5-dimethyl-6-isopropyl-5,6-dihydrouracil.

Als Komponente (c) der erfindungsgemässen Stoffgemische können die üblichen Härtungsmittel für Epoxidharze eingesetzt werden. Beispiele dafür sind:
Aliphatische, cycloaliphatische, aromatische und heterocyclische Amine, wie Bis(4-aminophenyl)methan, Anilin-Formaldehyd-Harze, Bis(4-aminophenyl)sulfon, Propan-1,3-diamin, Hexamethylendiamin, Diethylentriamin, Triethylentetramin, 2,2,4-Trimethylhexan-1,6-diamin, m-Xylylendiamin, Bis(4-aminocyclohexyl)methan, 2,2-Bis(4-aminocyclohexyl)propan und 3-Aminomethyl-3,5,5-trimethylcyclohexylamin (Isophorondiamin), Polyamino amide, wie beispielsweise solche aus aliphatischen Polyaminen und dimerisierten oder trimerisierten Fettsäuren, Polyphenole, wie Resorcin, Hydrochinon, 2,2-Bis(4-hydroxyphenyl)propan und Phenol-Aldehyd-Harze, Polythiole, wie die im Handel unter der Bezeichnung "Thiokole" erhältlichen Polythiole, Polycarbonsäuren und ihre Anhydride, wie zum Beispiel Phthalsäureanhydrid, Tetrahydrophthalsäureanhydrid, Hexahydrophthalsäureanhydrid, Hexachlorendomethylentetrahydrophthalsäureanhydrid, Pyromellitsäuredianhydrid, Benzophenon-3,3,4',4'-tetracarbonsäuredianhydrid, die Säuren der zuvorgenannten Anhydride sowie auch Isophthalsäure und Terephthalsäure. Es können auch katalytisch wirkende Härtungsmittel verwendet werden, wie beispielsweise Zinnsalze von Alkansäuren (zum Beispiel Zinnoctanoat), Friedel-Crafts-Katalysatoren, wie Bortrifluorid und Bortrichlorid und ihre Komplexe und Chelate, die durch Umsetzung mit Bortrifluorid mit zum Beispiel 1,3-Diketonen erhalten werden.

Die Menge des eingesetzten Härtungsmittels richtet sich nach der chemischen Natur des Härtungsmittels und nach den gewünschten Eigenschaften der härtbaren Mischung und des gehärteten Produktes. Die optimale Menge kann leicht ermittelt werden. Wenn das Härtungsmittel ein Amin ist, werden normalerweise 0,75 bis 1,25 Aequivalente Aminwasserstoff pro 1 Epoxidäquivalent eingesetzt. Wenn Polycarbonsäuren oder ihre Anhydride eingesetzt werden, verwendet man gewöhnlich 0,4 bis 1,1 Aequivalente Carboxylgruppe bzw. Anhydridgruppe pro 1 Aequivalent Epoxidgruppe. Bei der Verwendung von Polyphenolen als Härtungsmittel setzt man zweckmässig 0,75 bis 1,25 phenolische Hydroxylgruppen pro 1 Epoxidäquivalent ein.

Katalytisch wirkende Härtungsmittel werden allgemein in Mengen von 1 bis 40 Gewichtsteilen pro 100 Gewichtsteile Epoxidharz eingesetzt.

Gewünschtenfalls kann man den härtbaren Gemischen zur Herabsetzung der Viskosität aktive Verdünner, wie z.B. Styroloxid, Butylglycidylether, 2,2,4-Trimethylpentylglycidylether, Phenylglycidylether, Kresylglycidylether oder Glycidylester von synthetischen, hochverzweigten, in der Hauptsache tertiären aliphatischen Monocarbonsäuren, zusetzen.

Man kann bei der Härtung ausserdem Härtungsbeschleuniger einsetzen; solche Beschleuniger sind z.B. tertiäre Amine, deren Salze oder quaternäre Ammoniumverbindungen, z.B. Benzyldimethylamin, 2,4,6-Tris(dimethylaminomethyl)phenol, 1-Methylimidazol, 2-Ethyl-4-methylimidazol, 4-Aminopyridin, Tripentylammoniumphenolat; oder Alkalimetallalkoholate, wie z.B. Na-Alkoholate von 2,4-Dihydroxy-3-hydroxymethylpentan.
Die Härtung der erfindungsgemässen Mischungen wird zweckmässig im Temperaturintervall von 50°C bis 300°C, bevorzugt von 80-250°C, durchgeführt.

Man kann die Härtung in bekannter Weise auch zwei- oder mehrstufig durchführen, wobei die erste Härtungsstufe bei niedriger Temperatur und die Nachhärtung bei höherer Temperatur durchgeführt wird.

Die Härtung kann gewünschtenfalls auch derart in 2 Stufen erfolgen, dass die Härtungsreaktion zunächst vorzeitig abgebrochen bzw. die erste Stufe bei wenig erhöhter Temperatur durchgeführt wird, wobei ein noch schmelzbares und/oder lösliches, härtbares Vorkondensat (sogenannte "B-Stufe") aus der Epoxy-Komponente (a) und dem Härter (b) erhalten wird. Ein derartiges Vorkondensat kann z.B. zur Herstellung von "Prepregs", Pressmassen oder Sinterpulvern dienen.

Der Ausdruck "Härten", wie er hier gebraucht wird, bedeutet die Umwandlung der löslichen, entweder flüssigen oder schmelzbaren Polyepoxide in feste, unlösliche und unschmelzbare, dreidimensional vernetzte Produkte bzw. Werkstoffe, und zwar in der Regel unter gleichzeitiger Formgebung zu Formkörpern, wie Giesskörpern, Presskörpern und Schichtstoffen, zu Imprägnierungen, Beschichtungen, Lackfilmen oder Verklebungen.

Die erfindungsgemässen härtbaren Gemische können ferner geeignete Weichmacher, wie Dibutylphthalat, Dioctylphthalat oder Trikresylphthalat, enthalten.

Schliesslich können die erfindungsgemässen härtbaren Gemische vor der Härtung in irgendeiner Phase mit Streck-, Füll- und Verstärkungsmitteln, wie beispielsweise Steinkohlenteer, Bitumen, Textilfasern, Glasfasern, Asbestfasern, Borfasern, Kohlenstoff-Fasern, mineralischen Silikaten, Glimmer, Quarzmehl, Aluminiumoxidhydrat, Bentoniten, Kaolin, Kieselsäureaerogel oder Metallpulvern, z.B. Aluminiumpulver oder Eisenpulver, ferner mit Pigmenten und Farbstoffen, wie Russ, Oxidfarben, Titandioxid u.a. versetzt werden. Mann kann der härtbaren Gemischen ferner auch andere übliche Zusätze. z.B. Flammschutzmittel, wie Antimontrioxid,

Thixotropiemittel, Verlaufmittel ("flow control agents"), wie Silicone, Wachse oder Stearate (welche zum Teil auch als Formtrennmittel Anwendung finden), zusetzen.

Die Herstellung der erfindungsgemässen härtbaren Mischungen kann in üblicher Weise mit Hilfe bekannter Mischaggregate (Rührer, Kneter, Walzen etc.) erfolgen.

Die erfindungsgemässen härtbaren Epoxidharzmischungen finden ihren Einsatz vor allem auf den Gebieten des Oberflächenschutzes, der Elektrotechnik, der Laminierverfahren und im Bauwesen. Sie können in jeweils dem speziellen Anwendungszweck angepasster Formulierung, im ungefüllten oder gefüllten Zustand, als Anstrichmitel, Lacke, wie Sinterpulverlacke, als Pressmassen, Tauchharze, Giessharze, Spritzgussformulierungen, Imprägnierharze und Klebmittel, als Werkzeugharze, Laminierharze, Dichtungs- und Spachtelmassen, Bodenbelagsmassen und Bindemittel für mineralische Aggregate verwendet werden.

Die mit den erfindungsgemässen Verbindungen der Formel I hergestellten ausgehärteten Produkte zeichnen sich durch sehr gute thermische und mechanische Eigenschaften aus. Im Vergleich mit Produkten, welche ohne die Mitverwendung der erfindungsgemässen Orthokohlensäureester hergestellt wurden, weisen sie eine vergleichbar hohe Wärmeformbeständigkeit bei wesentlich höherer Biegefestigkeit und Schlagzähigkeit auf und zudem ist die sonst beobachtete Volumenverminderung während der Härtung reduziert bzw. ganz aufgehoben.

Die folgenden Beispiele erläutern die Erfindung näher.

Beispiel 1: Umsetzungsprodukt von 2,2-Dichlor-1,3-benzodioxol mit Glycerin

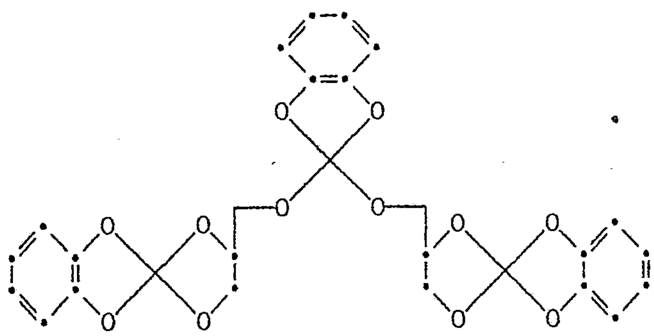

IV

30,8 g (0,335 Mol) Glycerin werden in 200 ml Pyridin und 100 ml Toluol in einer Apparatur bestehend aus einem 500 ml Dreihalskolben, Magnetrührer, Thermometer, Tropftrichter und Trockenrohr vorgelegt. Dazu werden unter Feuchtigkeitsausschluss bei 10-20° C 95,5 g (0,50 Mol) 2,2-Dichlor-1,3-benzodioxol zugetropft. Anschliessend rührt man über Nacht bei Raumtemperatur. Danach giesst man auf Eis und verdünnte HCl, fügt noch 200 ml Toluol zu, trennt die organische Phase ab und wäscht sie mit 1N HCl und gesättigter $NaHCO_3$-Lösung. Nach dem Trocknen über Natriumsulfat und Eindampfen am Rotationsverdampfer verbleiben 83 g (92 % d.Th.) eines hellgelben Oels.

| Elementaranalyse: | % C | % H |
|---|---|---|
| berechnet für $C_{27}H_{22}O_{12}$: | 60,11 | 3,30 |
| gefunden: | 60,55 | 4,29 |

Das Produkt hat ein mittels Gelpermeationschromatographie (in THF, Polystyrol als Standard) bestimmtes Molekulargewicht $\overline{M}_n = 592$ und $\overline{M}_w = 690$.

Beispiel 2: Umsetzungsprodukt von 2,2-Dichlor-1,3-benzodioxol mit 1,1,1-Trimethylolpropan

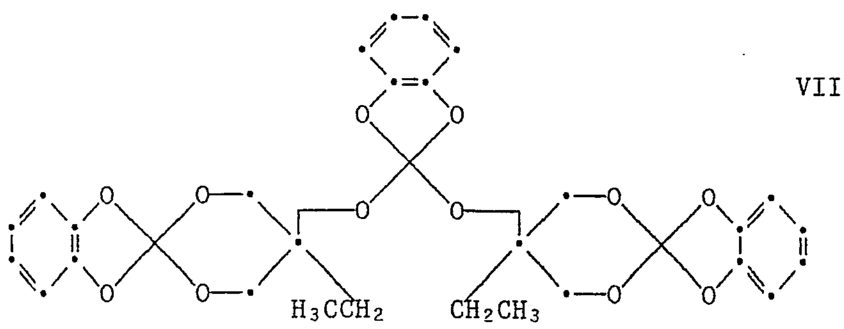

VII

40,0 g (0,300 Mol) 1,1,1-Trimethylolpropan werden in 120 ml Pyridin und 200 ml Methylenchlorid in einer Apparatur bestehend aus einem 1000 ml Dreihalsrundkolben, Magnetrührer, Thermometer, Tropftrichter und Trockenrohr vorgelegt. Dazu werden unter Feuchtigkeitsausschluss bei 10-20°C 85,4 g (0,45 Mol) 2,2-Dichlor-1,3-benzodioxol zugetropft. Anschliessend rührt man über Nacht bei Raumtemperatur. Danach giesst man auf Eis und verdünnte Salzsäure, trennt die organische Phase ab und wäscht sie mit 1N HCl und gesättigter $NaHCO_3$-Lösung. Nach dem Trocknen über Natriumsulfat und Eindampfen am Rotationsverdampfer erhält man 80,8 g (87% d.Th.) farblose Kristalle mit Fp. 163-166°C.

| Elementaranalyse: | % C | % H |
|---|---|---|
| berechnet für $C_{33}H_{34}O_{12}$: | 63,66 | 5,50 |
| gefunden: | 63,62 | 5,50 |

Beispiel 3: Umsetzungsprodukt von 2,2-Dichlor-1,3-benzodioxol mit 1,2,4-Butantriol

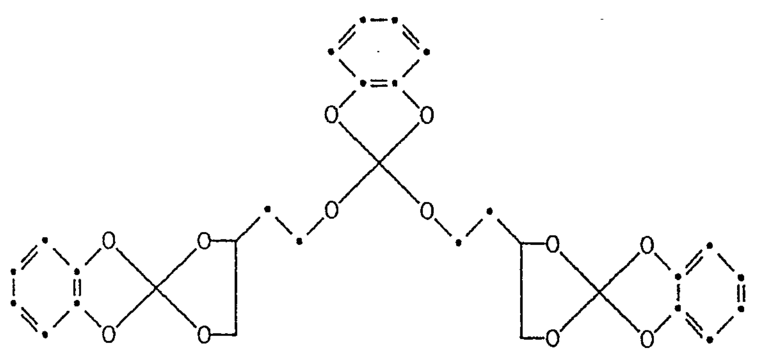

VIII

31,8 g (0,300 Mol) 1,2,4-Butantriol werden in 120 ml Pyridin und 200 ml Methylenchlorid in einer Apparatur bestehend aus einem 500 ml Dreihalsrundkolben, Magnetrührer, Thermometer, Tropftrichter und Trockenrohr vorgelegt. Dazu werden unter Feuchtigkeitsausschluss bei 10-20°C 85,4 g (0,45 Mol) 2,2-Dichlor-1,3-benzodioxol zugetropft. Anschliessend rührt man über Nacht bei Raumtemperatur. Danach giesst man auf Eis und verdünnte Salzsäure, trennt die organische Phase ab und wäscht sie mit 1N HCl und gesättigter $NaHCO_3$-Lösung. Nach dem Trocknen über Natriumsulfat und Eindampfen am Rotationsverdampfer verbleiben 86 g gelbliches Oel.

| Elementaranalyse: | % C | % H |
|---|---|---|
| berechnet für $C_{29}H_{26}O_{12}$: | 61,48 | 4,63 |
| gefunden: | 60,55 | 4,63 |

Beispiel 4: Anwendung des Produkts aus Beispiel in einem härtbaren Stoffgemisch

Die härtbaren Stoffgemische A und B werden durch Zusammenmischen der Komponenten bei Raumtemperatur hergestellt, in Formen gegossen und anschliessend während 4,5 Stunden bei 100°C und 1 Stunde bei 150°C gehärtet. Die gehärteten Formkörper haben die in Tabelle 1 angegebenen Eigenschaften.

Tabelle 1

| Stoffgemisch | Tg (DSC) (°C) | Biege-festigkeit DIN 53452 ($N/mm^2$) | Schlagbiege-zähigkeit DIN 53453 ($kJ/m^2$) |
|---|---|---|---|
| A<br>100 g eines Bisphenol A Diglycidylethers mit einem Epoxidgehalt von 5,4 Aequ./kg<br>13 g m-Phenylendiamin<br>0,14 g $CH_3CH_2NH_2 \cdot BF_3$<br>32 g Produkt gemäss Bsp. 1 | 112 | 184,0 | 13,1 |
| B<br>100 g eines Bisphenol A Diglycidylethers mit einem Epoxidgehalt von 5,4 Aequ./kg<br>9,8 g m-Phenylendiamin<br>0,11 g $CH_3CH_2NH_2 \cdot BF_3$ | 113 | 177,7 | 10,0 |

Aus Tabelle 1 ist ersichtlich, dass die mit dem erfindungsgemässen Stoffgemisch (A) hergestellten gehärteten Formteile bessere Eigenschaften aufweisen als Formteile, die mit dem gleichen Epoxidharz-Härter-System aber ohne einen Orthokohlensäureester der Formel I als Cokomponente hergestellt werden (B).

Beispiel 5: Umsetzungsprodukt von 2,2-Dichlor-1,3-benzodioxol mit 1,1,1-Trimethylolethan

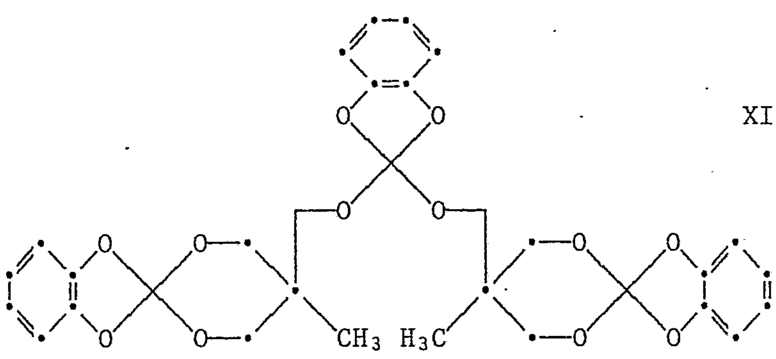

XI

39,2 g (0.33 Mol) 1,1,1-Trimethylolethan werden in 160 ml Pyridin und 200 ml Methylenchlorid in einer Apparatur bestehend aus einem 500 ml Dreihalsrundkolben, Magnetrührer, Thermometer, Tropftrichter und Trockenrohr vorgelegt. Dazu werden unter Feuchtigkeitsausschluss bei 10-20°C 93,4 g (0,50 Mol) 2,2-Dichlor-1,3-benzodioxol zugetropft. Anschliessend rührt man über Nacht bei Raumtemperatur. Danach giesst man auf Eis und verdünnte Salzsäure, trennt die organische Phase ab und wäscht sie mit 1N HCl und gesättigter $NaHCO_3$-Lösung. Nach dem Trocknen über Natriumsulfat und Eindampfen am Rotationsverdampfer verbleibt ein Rückstand von 92,8 g mit Schmelzpunkt von 165-168°C (farbloses Pulver).

| Elementaranalyse: | % C | % H |
|---|---|---|
| berechnet für $C_{31}H_{30}O_{12}$ | 62,62 | 5,09 |
| gefunden: | 62,09 | 5,10 |

Beispiel 6

Die härtbaren Stoffgemische C und D werden durch Zusammenmischen der Komponenten (Dreiwalzenstuhl) hergestellt, in Formen gegossen und anschliessend während 4,5 Stunden bei 100°C und 1 Stunde bei 150°C gehärtet. Die gehärteten Formkörper haben die in Tabelle 2 angegebenen Eigenschaften.

Tabelle 2

| Stoffgemisch | Tg(DSC) (°C) | Biegefestigkeit DIN 53452 ($N/mm^2$) | Schlagbiegezähigkeit DIN 53453 ($kJ/m^2$) | Kaltwasseraufnahme (4 Tage) (%) | Kochwasseraufnahme (1h) (%) |
|---|---|---|---|---|---|
| C<br>180 g eines Bisphenol A Diglycidylethers mit einem Epoxidgehalt von 5,4 Aequ./kg,<br>2 g $CH_3CH_2NH_2 \cdot BF_3$<br>20 g Produkt gemäss Beispiel 1 | 107 | 143 | 17,3 | 0,16 | 0,32 |
| D<br>200 g eines Bisphenol A Diglycidylethers mit einem Epoxidgehalt von 5,4 Aequ./kg<br>2 g $CH_3CH_2NH_2 \cdot BF_3$ | 107 | 136 | 14,9 | 0,21 | 0,39 |

Aus Tabelle 2 ist ersichtlich, dass die mit dem erfindungsgemässen Stoffgemisch (C) hergestellten gehärteten Formteile bessere Eigenschaften aufweisen als Formteile, die mit dem gleichen Epoxidharz-Härter-System aber ohne einen Orthokohlensäureester der Formel I als Cokomponente hergestellt werden (D).

**Patentansprüche**

1. Orthokohlensäureester der Formel I

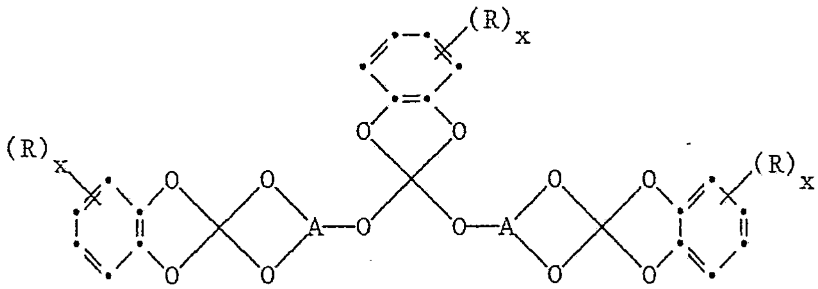

(I),

worin R $C_1$-$C_4$-Alkyl und x Null, 1 oder 2 bedeuten und A ein von einem aliphatischen Triol durch Weglassen der Hydroxylgruppen abgeleiteter dreiwertiger Rest ist, wobei die Hydroxylgruppen des Triols an verschiedene Kohlenstoffatome gebunden sind.

2. Orthokohlensäureester der Formel I nach Anspruch 1, worin x 1 und insbesondere Null bedeutet.

3. Orthokohlensäureester der Formel I nach Anspruch 1, worin A von einem 1,2,3-, 1,2,4- oder von einem 1,3,5-Triol abgeleitet ist.

4. Orthokohlensäureester der Formel I nach Anspruch 1, worin A ein 3 bis 24 Kohlenstoffatome enthaltender Rest ist.

5. Orthokohlensäureester der Formel I nach Anspruch 1, worin A ein Rest der Formel II

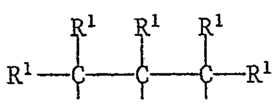

(II) ist,

ist,
wobei die Substituenten $R^1$ unabhängig voneinander eine $C_1$-$C_4$-Alkylgruppe oder vorzugsweise Wasserstoff bedeuten.

6. Orthokohlensäureester der Formel I nach Anspruch 1, worin A ein Rest der Formel III, IIIa oder IIIb

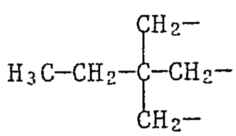

(III)

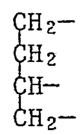

(IIIa)

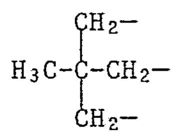

ist.

(IIIb)

ist.

7. Orthokohlensäureester nach Anspruch 1 der Formeln IV bis XI

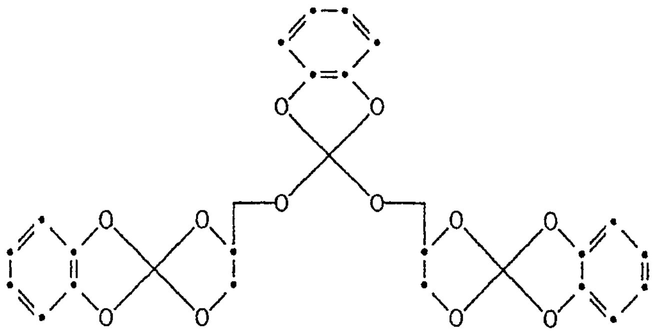

IV

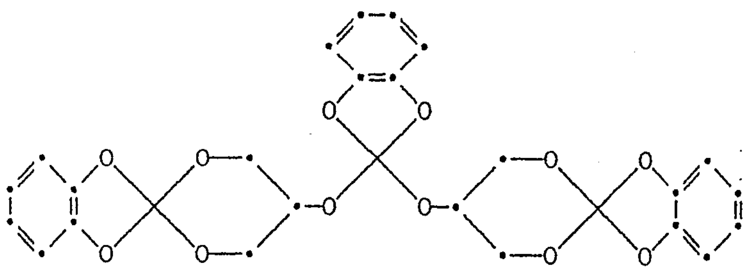

V

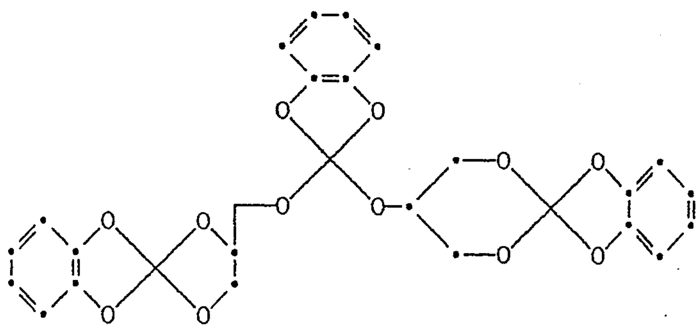

VI

VII

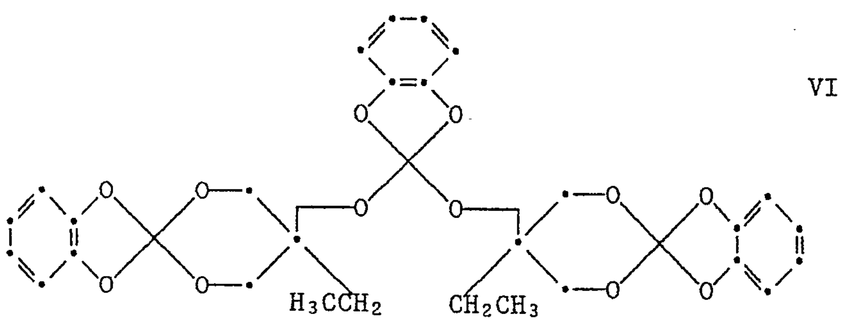

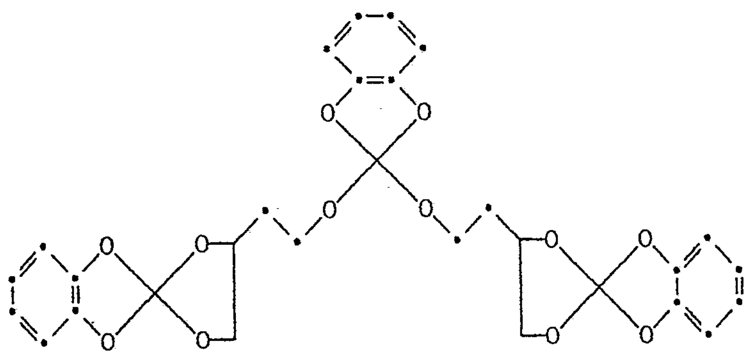

VIII

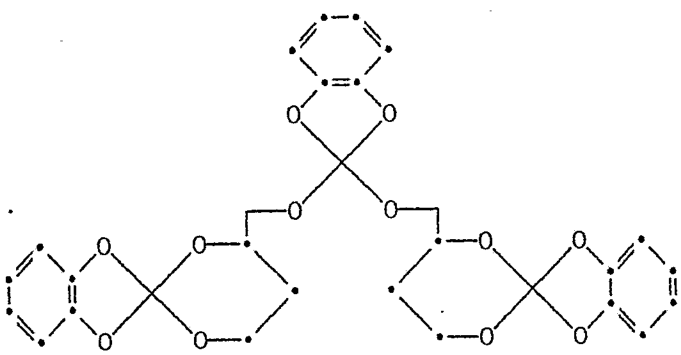

IX

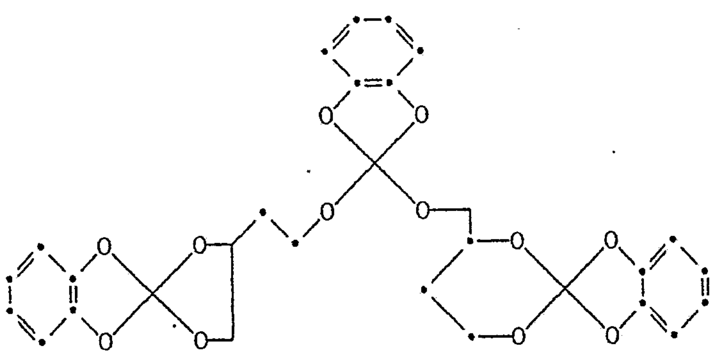

X

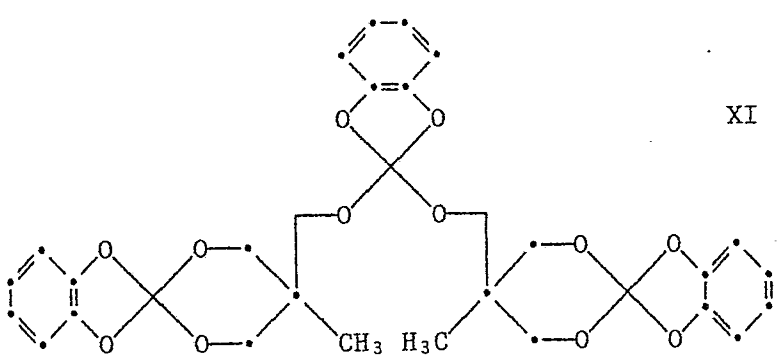

XI

8. Verfahren zur Herstellung von Orthokohlensäureestern der Formel I nach Anspruch 1 durch Umsetzung von drei Mol eines 2,2-Dichlor-1,3-benzodioxols der Formel

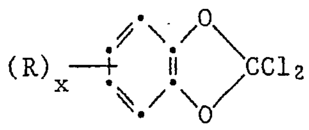

mit zwei Mol eines Triols der Formel $A(OH)_3$ in Gegenwart einer Base, wobei die Symbole R, x und A die im Anspruch 1 angegebene Bedeutung haben.

9. Härtbare Stoffgemische enthaltend
   (a) einen Orthokohlensäureester der Formel I nach Anspruch 1,
   (b) ein Epoxidharz und
   (c) einer Härter und gegebenenfalls einen Härtungskatalysator für das Epoxidharz.

10. Stoffgemische nach Anspruch 9, worin die Menge des Orthokohlensäureesters (a) 5 bis 50 Gewichtsteile pro 100 Gewichtsteile des Epoxidharzes (b) beträgt.

11. Verwendung der Orthokohlensäureester der Formel I nach Anspruch 1 als Cokomponente bei der Härtung von Epoxidharzen, zur Reduktion der während der Härtung von Epoxidharzen auftretenden Volumenverminderung.

**Patentansprüche für den folgenden Vertragsstaat:ES**

1. Verfahren zur Herstellung von Orthokohlensäureestern der Formel I

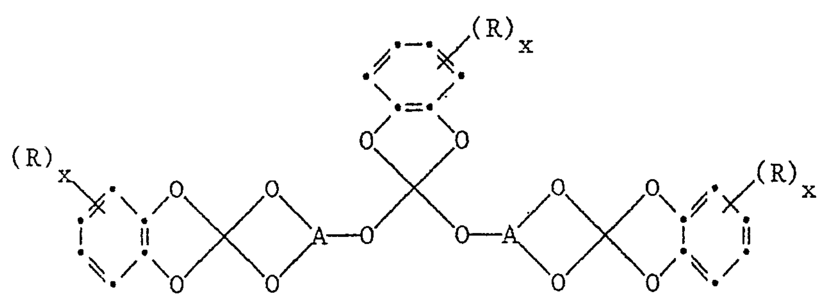

(I),

worin R $C_1$-$C_4$-Alkyl und x Null, 1 oder 2 bedeuten und A ein von einem aliphatischen Triol durch Weglassen der Hydroxylgruppen abgeleiteter dreiwertiger Rest ist, wobei die Hydroxylgruppen des Triols an verschiedene Kohlenstoffatome gebunden sind, durch Umsetzung von drei Mol eines 2,2-Dichlor-1,3-benzodioxols der Formel

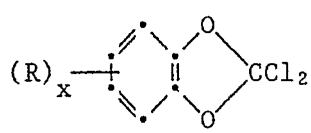

mit zwei Mol eines Triols der Formel $A(OH)_3$ in Gegenwart einer Base, wobei die Symbole R, x und A die oben angegebene Bedeutung haben.

2. Verfahren nach Anspruch 1, worin x 1 und insbesondere Null bedeutet.

3. Verfahren nach Anspruch 1, worin A von einem 1,2,3-, 1,2,4- oder von einem 1,3,5-Triol abgeleitet ist.

4. Verfahren nach Anspruch 1, worin A ein 3 bis 24 Kohlenstoffatome enthaltender Rest ist.

5. Verfahren nach Anspruch 1, worin A ein Rest der Formel II

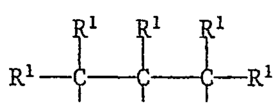

(II)

ist,

wobei die Substituenten $R^1$ unabhängig voneinander eine $C_1$-$C_4$-Alkylgruppe oder vorzugsweise Wasserstoff bedeuten.

6. Verfahren nach Anspruch 1, worin A ein Rest der Formel III, IIIa oder IIIb

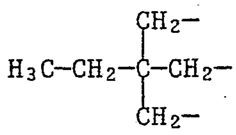
(III)

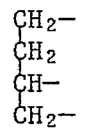
(IIIa)

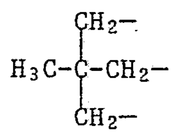
(IIIb)

ist.

7. Verfahren nach Anspruch 1 zur Herstellung von Orthokohlensäureestern der Formeln IV bis XI

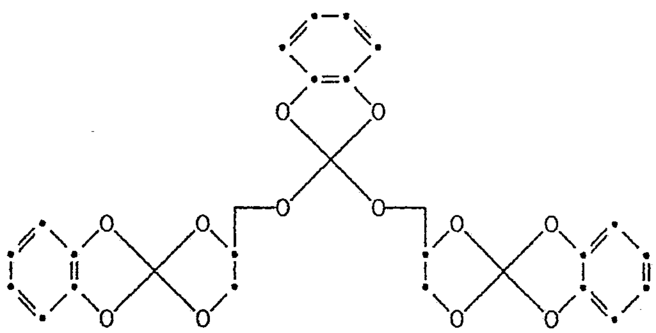
IV

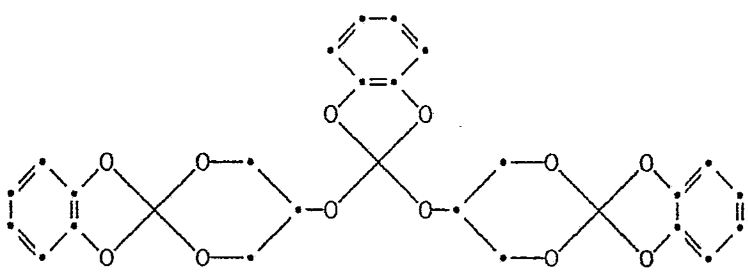
V

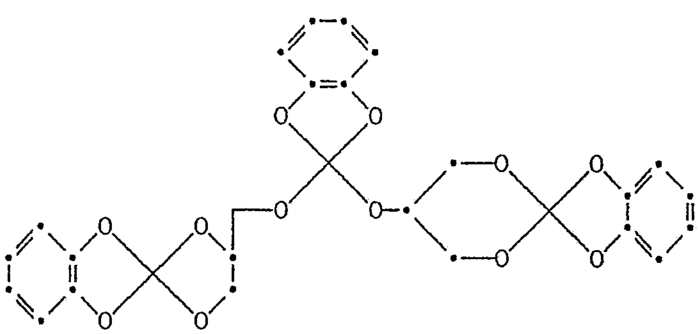
VI

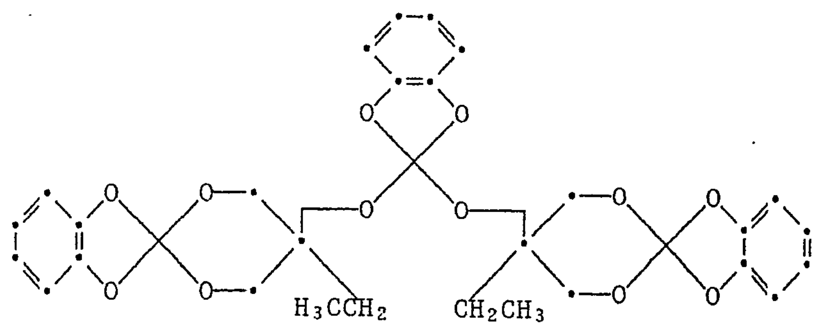

VII

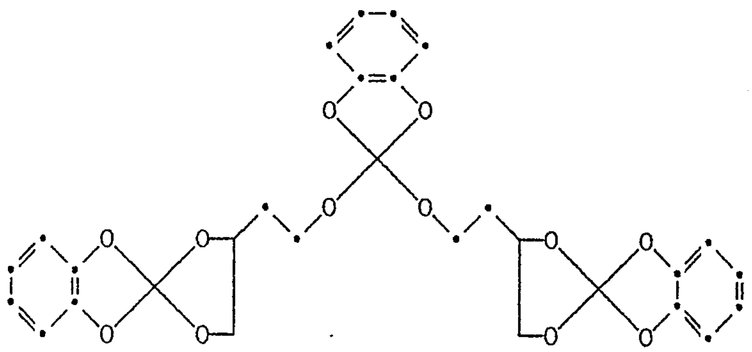

VIII

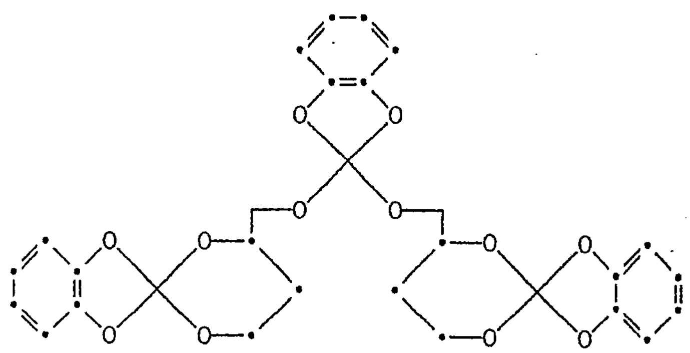

IX

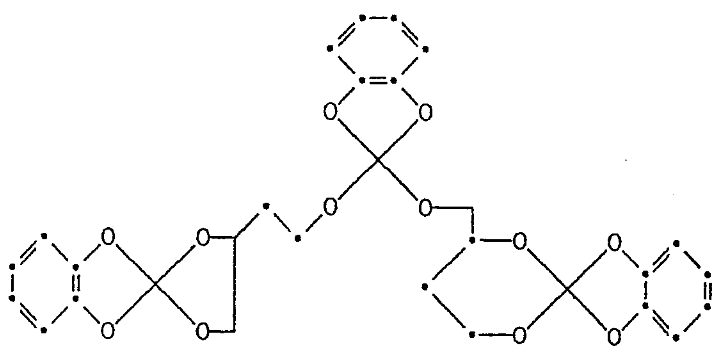

X

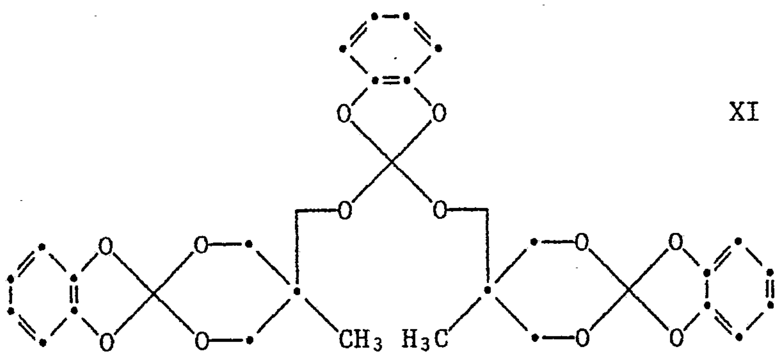

XI

8. Verwendung der Orthokohlensäureester der Formel I nach Anspruch 1 als Cokomponente bei der Härtung von Epoxidharzen, zur Reduktion der während der Härtung von Epoxidharzen auftretenden Volumenverminderung.